Europäisches Patentamt

European Patent Office

Office européen des brevets

Publication number: **0 300 740**
**A2**

# EUROPEAN PATENT APPLICATION

Application number: 88306612.8

Date of filing: 20.07.88

Int. Cl.⁴: **C12N 15/00 , C12P 21/00 ,
B01D 15/08 , A61M 1/36 ,
G01N 33/577 , G01N 33/68 ,
C12N 5/00 , //(C12P21/00,
C12R1:91)**

Priority: **20.07.87 NO 873017**

Date of publication of application:
**25.01.89 Bulletin 89/04**

Designated Contracting States:
**AT BE CH DE ES FR GB GR IT LI LU NL SE**

Applicant: **CEFEM A/S
P.O. Box 3570
N-7002 Trondheim(NO)**

Inventor: **Iversen, Ole-Jan
Department of Microbiology Regional
Hospital
7006 Trondheim(NO)**
Inventor: **Bergh, Kare
Department of Microbiology Regional
Hospital
7006 Trondheim(NO)**

Representative: **Wain, Christopher Paul et al
A.A. THORNTON & CO. Northumberland
House 303-306 High Holborn
London WC1V 7LE(GB)**

Monoclonal antibodies to complement C5a.

Monoclonal antibodies against complement factor C5a are made by:
  a) immune stimulation of spleen cells with C5a;
  b) hybridization of the immunized spleen cells and myeloma cells;
  c) selection of hybridoma cells for the production of antibodies against C5a; and
  d) implantation of the hybridoma cells in the peritoneal cavity of animals of the same species from which the spleen cells and the myeloma cells were isolated, to produce the antibodies.
  The antibodies can be linked to an immunoadsorbent material and be applied in the ELISA method, optionally in kit form, for the detection and quantification of C5a.

EP 0 300 740 A2

## MONOCLONAL ANTIBODIES TO COMPLEMENT C5a

The present invention concerns monoclonal antibodies against complement factor C5a, their production, the application for the removal of C5a from biological fluids, an immunosorbent material to which such antibodies have been bound and an ELISA method for the detection and quantification of C5a by use of (such) monoclonal antibodies, plus a kit for use in the mentioned ELISA method.

In Norwegian patent no. 85.2705 animal monoclonal antibodies against human C1q are described together with their application in the detection and quantification of C1q in a solution. Furthermore a kit for such use is described.

Norwegian patent no. 85.2426 describes monoclonal antibodies against endotoxin from Gram-negative bacteria. This publication also describes the immunological detection of Gram-negative bacteria or endotoxin from Gram-negative bacteria and the use of the monoclonal antibodies bound to a matrix for the cleansing of blood for Gram-negative bacteria or endotoxin from Gram-negative bacteria, i.e. therapeutic applications.

The complement system consists of a complex series of proteins in plasma, of which many are proteases. It is phylogenetic older than the specific immune system, and is probably developed as a important defence mechanism against microorganisms. There are two main ways of activating the complement system, termed the classical and the alternative pathway, respectively. The classical activation is usually initiated by antibodies (IgG and IgM) bound to antigen. Alternative activation may be triggered by microbial structures in the absence of antibodies, e. g. endotoxin and zymosan. Activation may also be triggered on the surface of non-biological material. Both pathways converge at the level of complement factor C3 in a cascade reaction that leads to the assembly of the subsequent complement proteins in the cascade to the lytic membrane attack complex (MAC) (a complex of proteins assembled on membranes and which have the capacity to destroy biological membranes). During reactions of the complement system low-molecular weight peptides are split from the N-terminal end of the alpha chain of C3 and C5, respectively. These peptides termed anaphylatoxins (C3a and C5a) are highly potent mediators of inflammation. Histamine released from mast cells by the anaphylatoxins causes increased vascular permeability and smooth muscle contraction. C5a is chemotactic (i.e. causes a non-directional migration) to neutrophil granulocytes and causes the aggregation of the same cells. Other effects of the anaphylatoxins are the increase in metabolic acitivity in neutrophil granulocytes and the release of toxic oxygen radicals and leukotrienes. Due to these various biological effects the anaphylatoxins, in particular C5a, have been regarded as possible pathogenetic factors in disease conditions like the adult respiratory distress syndrome (ARDS), and in complications occurring during hemodialysis.

As mentioned above, when blood is brought into contact with non-biological substances, the complement system may be activated leading to the release of C5a which may cause serious biological effects in patients. Thus, it has been the purpose of this invention to produce monoclonal antibodies which can bind complement factor C5a both to detect and to quantify the production of C5a under various circumstances, e. g. by extracorporal blood cleansing. Furthermore we wish to develop a method and a device for the removal of complement factor C5a from biological fluids, in particular blood.

According to the present invention production of monoclonal antibodies against complement factor C5a is obtained by

a) immune stimulation of spleen cells with C5a,

b) hybridization of immunized spleen cells and myeloma cells.

c) selection of hybridoma cells for the production of antibodies against C5a and

d) for the production of antibodies, hybridoma cells may be implanted into the peritoneal cavity of animals of the same species from which spleen cells and myeloma cells were isolated.

The antibodies are isolated from the ascitic fluids by well known methods as described below.

The monoclonal antibodies against C5a can be coupled to matrix substances by various coupling agents e. g. CNBr.

According to the present invention the removal of complementfactor C5a from biological fluids will be obtained when fluids containing C5a are brought in contact with a material to which monoclonal antibodies against C5a are bound.

Sepharose or monodisperse particles are to be preferably used as matrix materials. Among the monodisperse particles, monodisperse polystyrene particles are preferred.

As indicated above, an immunoadsorption material for the binding of complement factor C5a constitutes a matrix to which monoclonal antibodies against C5a are covalent bound as ligands. The preferential matrix

2

materials are mentioned above.

The problem outlined above concerning the detection and quantification of C5a may according to the present invention be detected and quantified as folows:

a) micro-ELISA-wells are coated with monoclonal antibody against C5a,

b) when free binding sites in the wells have been blocked by a suitable material, the wells are supplied with the biological fluid which is suspected to contain C5a, the C5a-antigen is then recognized by the antibody in the wells and bound to it by structural specificity,

c) unbound material is washed away,

d) labelled monoclonal antibody (e.g. biotin-conjugated or enzyme-conjugated) with specificity for other structures on C5a than that of the coating antibodies are then added to the wells,

e) unbound material is washed away,

f) if the labelled monoclonal antibody is labelled with biotin, then streptavidin with high affinity for biotin is added,

g) the wells are washed thoroughly,

h) the bound enzyme is demonstrated by the addition of substrate for the conjugated enzyme,

i) the reaction product formed is observed qualitatively or quantified.

The present inventors have used the following method for the production and isolation of C5a from rabbit:

a) Activation of rabbit-serum by using Zymosan A (Sigma).

b) Activated serum is applied on a CM-Sephadex C-25 column equilibrated in 0.1M Ac-buffer pH 6.0, and chemotacted factor is eluted with a linear gradient (0.15 to 1.15M) NaCl in the same buffer.

c) Eluted fractions containing chemotactic acitivity are applied on a DEAE-Sephadex A-50 column equilibrated in an 0.02 M phosphate-buffer pH 7.0, and all chemotactic activity passed the anion exchange column. Thus C5a is present in the eluate. This is used as vaccine when immunizing mice.

The production of monoclonal antibodies is performed according to the method described by Køhler and Milstein: "Continuous cultures of fused cells secreting antibody of predefined specificity." Nature 256, 495-497, 1975 and Goding: "Antibody production by hybridomas." J. Immunol. Methods, 39, 285-308, 1980. The selection of hybridoma cultures was based on: 1) ELISA (using the vaccine fraction and the vaccine fraction purified by Isolelectric focusing as antigen), 2) immunoblot with the vaccine fraction on nitrocellulose membranes, 3) inhibition of chemotactic activity. The specificity of the antibodies is justified by the abolishment of chemotactic activity. By affinity chromatography C5a is bound to CNBr-activated Sepharose 4B columns with monoclonal antibody as ligand. C5a can be eluted at low pH as demonstrated by chemotactic activity and immunological reactivity in the eluate.

Based on this principle a series of monoclonal antibodies against C5a with specificities for various parts of the C5a molecule were produced.

The production of murine monoclonal antibodies against rabbit chemotactic peptide C5a is described below.

MATERIALS AND METHODS.

Animals.

Chinchilla rabbits and female BALB-c mice were purchased from Statens Institutt for Folkehelse, Oslo, Norway.

Preparation of purified human PMNs.

Blood from healthy human volunteers was drawn in sterile vacutainers containing 15 units of sodium heparin per ml (Becton Dickinson, Meylan Cedex, France). Purification of PMNs was obtained by the method of Bøyum (1968). The final concentration of the purified PMNs was adjusted to $5 \times 10^7$ · ml saline.

Chemotaxis assay.

Neutrophil chemotaxis was assessed by the under agarose method described by Nelson et al. (1975) with minor modifications. 1 g agarose ( Litex agarose for gel electrophoresis, Type No HSA,Litex. Denmark) in 45 ml sterile. distilled water was heated for 30 min at 100 °C. The solution was cooled to 56 °C, mixed with an equal volume of warmed (56 °C) 2 x Minimal Essential Medium Eagle (MEM) ( Flow Lab.. Herts. England). 10 ml heat-inactivated human serum and 37.5 mg sodium bicarbonate then was added. The warm solution was plated into 50 x 15 mm cell culture dish (Nunclon delta SI, Nunc. Roskilde. Denmark. 8 ml dish). As different human sera may contain varying amounts of inhibitory factors. all experiments were performed using frozen aliquots of serum obtained from one donor.

In each dish, six series of three wells. 2.5 mm in diameter and spaced 2.5 mm apart. were cut using a template and a punch. 10 $\mu$l of the purified PMN solution (i.e. $5 \times 10^5$ cells) was added to the centre well. The outer well received 10 $\mu$l of the sample to be investigated for chemotactic factor. and the inner well received 10 $\mu$l of MEM. The dishes were incubated for two hours at 37 °C in a humidified atmosphere containing 5 % $CO_2$.

The migratory distance of the PMNs was measured by in situ microscopic examination using an inverted microscope and a calibrated ocular micrometer. The respective migratory distances were defined as the distance from the margin of the centre well to the furthest point where 3 cells were aligned in the same plane and parallel to margin of centre well ; A: directed migration toward the chemoattractant or the outer well, B: random migration towards the inner well containing MEM. The chemotactic index is calculated as A  B.

Activation of serum.

Blood was drawn from the marginal ear vein of conscious rabbits (small volumes) or through cardiac puncture of anaesthetized rabbits into siliconized vacutainers (Becton Dickinson). After clotting the serum was centrifuged for 15 min at 600 g. Zymosan A ( Sigma Chemical Co,St. Louis,Mo., USA) in physiological saline was added to serum to a final concentration of 2 or 5 mg Zymosan A per ml serum. The control serum received an equal amount of saline. The serum was incubated at 37 °C for 2 hours under intermittent vigourous shaking. Zymosan particles were removed by centrifugation for 30 min at 800 g. The supernates were removed and subjected to further purification procedures or tested for chemotactic activity in the crude zymosan-activated serum (ZAS).

Preparation of purified zymosan-induced chemotactic factor.

The chromatography procedures described below were performed at room temperature. Cation exchange chromatography was done as described by Williams and Jose (1981) except for different conditions of elution. Serum (volume from 10 to 80 ml) was acidified to pH 6.0 by slowly adding 0.2 M sodium acetate buffer pH 3.8. The serum was applied to a column ( 1.5 x 4.0 cm) of carboxymethyl (CM)-Sephadex C-25 (Pharmacia Fine Chemical AB, Uppsala. Sweden) equilibrated in a mixture of isotonic saline and 0.1 M sodium acetate buffer pH 6.0 at a ratio of 5 : 1. Bound material then was eluted with a 30 ml linear NaCl gradient ranging from 0.15 M to 1.15 M in the equilibration buffer. 1 ml fractions were collected and tested for chemotactic activity. Fractions containing chemotactic activity were pooled and concentrated to one ml by ultrafiltration using a YM-5 filter ( Diaflo ultrafilters, Amicon Ireland Ltd,Limerick, Ireland). Ion exchange was performed on a 10.0 x 0.8 cm Sephadex G-25 column equilibrated in 0.02 M sodium phosphate buffer pH 7.2. before the material was applied to a 5.0 x 0.8 cm DEAE-Sephadex A-50 column equilibrated in 0.02 M sodium phosphate buffer pH 7.2. Material passing through the anion exchange column without binding to it was collected and tested for chemotactic activity, or stored at - 20 °C, as purified zymosan-induced chemotactic factor. Iso-electric focusing of the purified chemotactic factor was done ( for 24 hours at 1200 V) in a sucrose gradient ranging from 5 to 50 % (w/v) containing 1 % ampholyte solution ( Ampholine pH 3.5 - 10. LKB. Bromma. Sweden). The fractions, 1.8 ml each, were harvested by means of a peristaltic pump and were used as antigen in ELISA, or stored at -20 °C.

SDS-PAGE was performed according to the method of Laemmli (1970). Standard proteins (SDS-PAGE Standard Low Molecular Weight. Bio-Rad Laboratories. Richmond, CA, USA ) were run simultaneously with the samples. The gels were stained with Coomassie Brilliant Blue G (Sigma).

## Immunoblot.

After SDS-PAGE the gels were washed in 25 mM sodiumphosphate buffer pH 6.5, containing 0.1 % SDS ( 2 x 20 min). The samples were electrophoretically transferred(16 hours at 25 V) to nitrocellulose membrane (Bio-Rad ) in 25 mM sodium phosphate buffer pH 6.5. The nitrocellulose membrane was cut into strips and soaked into PBS containing 3 % Tween 20 (Sigma) for 30 minutes. The strips were incubated for two hours with culture supernates from hybridoma cultures diluted 1 : 10 in PBS containing 0.05 % Tween 20 (PBS-Tween) or with murine ascitic fluid diluted 1 : 1000 in PBS-Tween. All steps were followed by thorough washing with PBS-Tween. The binding of antibodies was detected by incubating the strips with peroxidase-conjugated rabbit anti-mouse immunoglobulins (Dakopatts a/s, Glostrup, Denmark) diluted 1 : 1000 in PBS-Tween. Finally, substrate orthophenylenediamine in 0.1 M citrate-phosphate buffer pH 5.0 was added.

## ELISA procedure

Linbro microtitration plates ( Flow) were used during all experiments. The wells were coated overnight at 20 $^\circ$C with antigen in 0.1 M carbonate buffer pH 9.6. The blocking procedure, washing, administration of culture supernates or ascitic fluid and peroxidase-conjugated antibodies were as described above for immunoblot. Ortho-phenylenediamine (OPD) was added as substrate, and colour development was terminated after 30 minutes by adding 2 M $H_2SO_4$ (Engwall 1980). The optical density at 492 nm was read in a Titertek multiscan spectrophotometer (Flow ).

## Production of monoclonal antibodies.

0.25 ml purified chemotactic factor (obtained from 6.5 ml serum) was emulsified in 0.25 ml Freund's complete adjuvant (Behringwerke AG, Marburg, W. Germany ) and injected intraperitoneally (ip) into mice. Four weeks later, an equal amount of antigen emulsified in Freund's incomplete adjuvant (Behringwerke) was given ip. A final dose of 0.1 ml antigen, dialyzed against saline, was injected intravenously in the tail two weeks later.

Three days later the spleen cells from one mouse were mixed with myeloma cells ( Sp2/0-Ag-14, from Flow) at a ratio of 4:1. The cells were fused as described by Oi & Herzenberg (1980), using 50 % polyethylene glycol 1000 (BDH, Chemicals Ltd,Poole, England) as a fusion agent ( Davidson et al.,1976). Hybrid cells were selected by cultivation in HAT-medium for two weeks in five 96-well tissue culture plates ( Nunclon 96 F, Nunc). The HAT-medium consisted of Dulbecco's modified Eagle medium (DMEM, Flow) supplemented with 15 % heat-inactivated foetal calf serum (Sera-Lab Ltd., Sussex, England), 292 mg/l L-glutamine (Flow), 100 mg/l gentamycin (Gibco Ltd.,Scotland), 1 mg/l amphotericin B (Gibco) and HAT (13.61 mg/l hypoxanthine, 0.18 mg/l aminopterine and 3.88 mg/l thymidine, from Flow). A Steri-Cult CO$_2$-incubator (Forma Scientific,USA) with 7 % CO$_2$, 95 % relative humidity and a temperature 37.0 $^\circ$C was used for the cultivation of the cells. After 14 days the cultures were screened for antibody production by ELISA using the purified chemotactic factor diluted 1 in 200 as antigen (corresponding to 12.5 microlitres serum per well). A selected number of 22 positive cultures were tested for inhibition of chemotactic activity as described above. According to the results obtained, seven cultures were cloned by limiting dilution ( De Blas et al.,1981) in 96-well tissue culture plates with a feeder layer of spleen cells (2x20$^5$ per well) from normal BALB/c-mice. The cells were grown in HT-medium (HAT-medium devoid of aminopterine, Flow). After eight to ten days the cultures were once again screened by ELISA and recloned, whereafter positive single-clone cultures were propagated in tissue culture flasks (Nunc).

Six single-clone cultures were grown as ascitic tumours in pristane- (2,6,10,14-tetramethyl pentadecane, Janssen Chimica, Belgium) primed mice. Debris and fat were removed from ascitic fluids by centrifugation and treatment with Trichlorfluroethan (Lipoclean, Behringwerke,). The fluids were frozen at -20 $^\circ$C in aliquots.

Immunoadsorption.

Monoclonal antibodies were purified from ascitic fluid by ion exchange chromatography on a DEAE-Sephacel column or by precipitation in ammonium sulphate. Purified monoclonal antibodies were coupled to CNBr-activated Sepharose 4B (Pharmacia ) according to the instructions of the manufacturer. Briefly. IgG (purified from one ml asctitic fluid) was incubated for 2 h at room temperature with 0.5 - 1.0 g CN-Br activated Sepharose 4B (preswollen and washed in 1 mM HCl) in 0.1 M bicarbonate buffer pH 8.3 containing 0.5 M NaCl. After the coupling, residual active groups were blocked by the addition of 0.5 M Glycin for 2 h. The gel was then washed repeatedly with 0.1 M acetate buffer pH 4.0 containing 0.5 M NaCl and the coupling buffer.

RESULTS.

Chemotactic activity and partial purification of C5a.

Cation exchange chromatography.

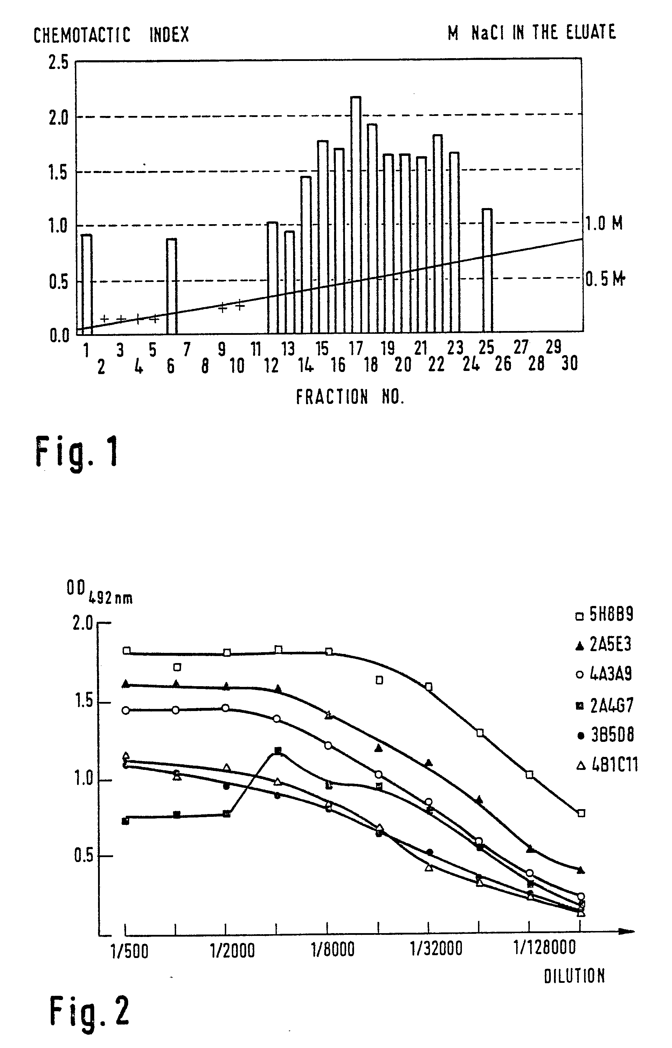

When volumes of rabbit serum (activated with Zymosan A 5 mg/ml) not exceeding 120 ml were applied to the CM-Sephadex column, all chemotactic activity was retained. It could be eluted from the column by a gradient of NaCl, typically beginning at 0.35-0.40 M NaCl. A typical result is shown in figure 1. Adjustment of the pH of the equilibrating buffer to 7.2, resulted in measurable chemotactic activity appearing in the effluent. SDS-PAGE of the fraction containing maximum chemotactic activity, showed at least 11 protein bands by staining with Coomassie brilliant blue. Three proteins with an estimated molecular weight less than 18 kD were regularly noted.

Anion exchange chromatography.

All chemotactic acitivity passed through the anion exchange column DEAE-Sephadex A-50 without binding to it. Several proteins eluted from CM-Sephadex were in this way removed, while the chemotactic activity was conserved.

Isoelectric focusing.

Fractions covering the pH range from 10.0 to 3.5 were examined, but no measurable chemotactic activity was detected.

Attempts to reestablish a physiological pH and ionic strength were insufficient with respect to detect chemotactic activity. A matter of concern is that prior to isoelectric focusing, the samples had to be dialyzed, and that the dialysis for as short as one hour led to 50 - 90 % loss of chemotactic activity (data not shown).

Monoclonal antibodies.

The fusion procedure, as described. resulted in more than 450 hybrid clones. Screening the culture wells by ELISA, 73 wells yielded an optical density above 0.8. Immunoblot analysis using the culture supernates from these 73 wells, showed that 25 wells were positive by the reaction with an antigen with a molecular weight less than 18 kD. Several of these were positive reacting with two low-molecular weight antigens. 7 cultures reacting with only one antigen with an estimated molecular weight of 15 kD, and which in preliminary tests reduced the chemotactic activity in ZAS, were cloned. ELISA positive clones were recloned, and six ELISA positive monoclones were grown as ascitic tumors in mice.

These six monoclonal antibodies were all high-titre antibodies using either fractions from CM-Sephadex.

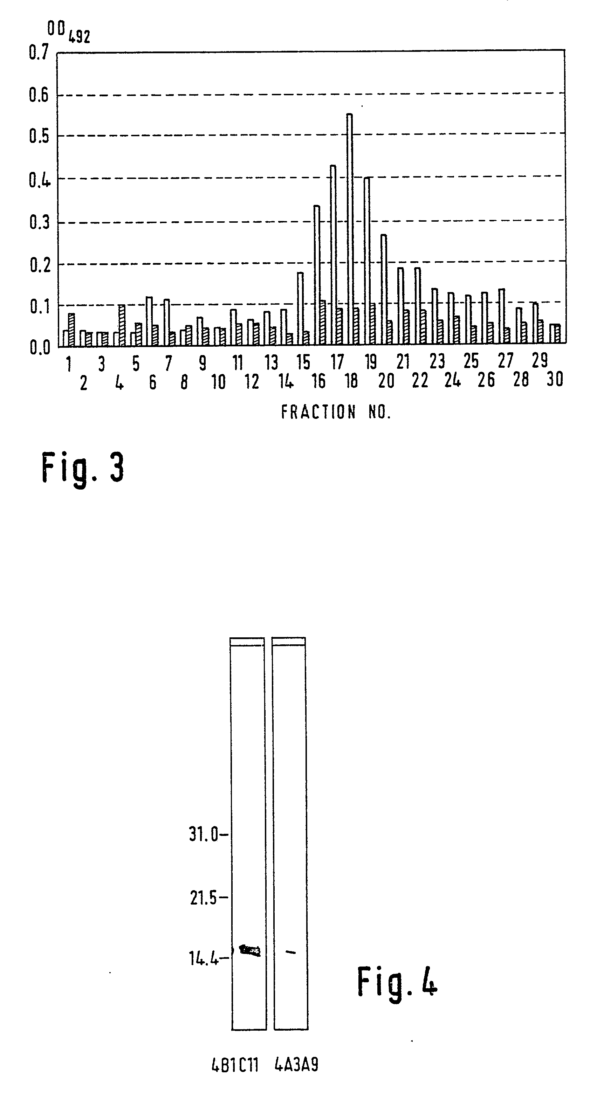

CM-Sephadex + DEAE-Sephadec or IEF as coating antigen. Figure 2 showns an example of the titration curve. Further support for the speci ficity of these antibodies against a component enriched by complement activation, was obtained from the experiments using eluted fractions from CM-Sephadex after the application of ZAS or a heat-inactiveated control serum, respectively (figure 3).

Immunoblot analysis using monoclonal antibodies.

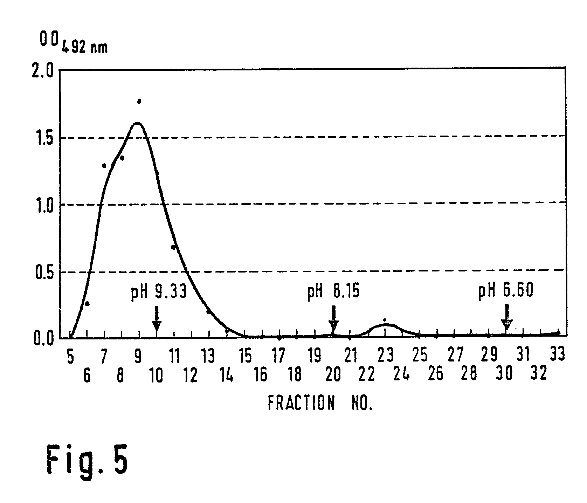

As shown in figure 4, the monoclonal antibodies were directed against an antigen with an estimated molecular weight of 15 kD.

Inhibition of chemotactic activity using monoclonal antibodies.

The incorporation of monoclonal antibodies into the agarose resulted in the complete or a partial abrogation of the chemotactic activity in ZAS. Table I shows the results obtained by the individual antibodies. When 5 microliters of ZAS was applied to the outer well of the agarose dish together with 5 microliters of monoclonal antibody (3B5D8, 4B1C11,2A5E3 or 4A3A9), a complete abrogation of the chemotactic activity was achieved (data not shown).

TABLE I

| Monoclonal antibody | A:directional migration | B:random migration | Chemotactic index A:B |
|---|---|---|---|
| 4B1C11 | 629 um | 708 um | 0.89 |
| 4A3A9 | 925 | 670 | 1.38 |
| 3B5D8 | 462 | 454 | 1.02 |
| 2A5E3 | 1008 | 608 | 1.66 |
| 2A4G7 | 470 | 483 | 0.97 |
| 5H8B9 | 720 | 700 | 1.03 |
| 2-1A10C8 | 1108 | 521 | 2.13 |
| SP 2/0 AG 14 | 1390 | 550 | 2.53 |

1 % ascitic fluid was incorporated into the agarose. The chemoattractant in these experiments were ZAS. Each result is the mean of six measurements. Monoclonal 2-1A10C8 is directed against a human glycoprotein and is included as a control by incorporating a irrelevant monoclonal antibody into the agarose. Sp 2/0 AG 14 is ascitic fluid produced by the myeloma cell line without a fusion with a spleen cell.

ELISA of ZAS isoelectric focusing.

Although no chemotactic activity was detected in material after isoelectric focusing, monoclonal antibodies detected the antigen in fractions harvested from the column after IEF (figure 5).

Immunoadsorption of chemotactic activity by monoclonal antibodies.

The immunoadsorbent columns efficiently bound the chemotactic activity in ZAS. Table II shows the result of ELISA and chemotaxis assay in fractions eluted from immunosorbent column using 2A5E3 as ligand. Immunoblot analysis of the acid eluted fraction containing chemotactic activity did show a protein band with an estimated molecular weight of approximately 15 kD (result not shown).

TABLE II

| PROFILE OF ELUTED FRACTIONS OF ZAS FROM CNBr-SEPHAROSE WITH 0.1 M GLYCINE-HCl pH 2.6 | | |
|---|---|---|
| Fraction no | ELISA OD $_{492}$ nm | Chemotactic index |
| 1 | 0.045 | 0.99 |
| 2 | 0.028 | 1.05 |
| 3 | 0.043 | 1.02 |
| 4 | 0.018 | 1.02 |
| 5 | 0.037 | 1.07 |
| 6 | 0.055 | 1.09 |
| 7 | 0.325 | 1.51 |
| 8 | 0.365 | 1.31 |
| 9 | 0.460 | 1.60 |
| 10 | 0.015 | 1.12 |

Monoclonal antibody 2A5E3 was used as ligand in this experiment. 4 ml of ZAS was applied to the immunosorbent column. One ml fractions were collected. Fractions no 1-2 were collected while ZAS was passing through the column. Fractions no 3-6 were collected while washing the column with PBS. 2 ml of 0.1 M Glycine-HCl pH 2.6 were applied corresponding to the start of fraction no 7, followed by washing with PBS. For the ELISA the fractions were diluted 1:50 in 0.1 M carbonate buffer pH 9.6. Monoclonal antibody 4A3A9 was used in the ELISA diluted 1:2000.

In the accompanying drawings:

Fig. 1 shows CM.Sephadex Chromatography of Zymosan-activated serum (28ml). The column was eluted with a 30 ml linear NaCl gradient.

Fig. 2 shows titration curves of optical density (at 492nm) against dilution for six monoclonal antibodies.

Fig. 3 shows the Elisa-CM-Sephadex chromatography of ZAS vs heat-inactivated serum (27ml). MAb 5-4A3A9 was diluted 1:20,000. The optical densities were measured at 492 nm.

Fig. 4 is a Western immunoblot analysis of 4B1C11 and 4A3A9 (diluted 1 in 1000) reacted with purified chemotactic factor separated by SDS-PAGE (12% acrylamide). The molecular weight standards were run simultaneously.

Fig. 5 shows isoelectric focussing - Elisa. Purified chemotactic factor from 70 ml zymosan-activated serum. Optical density was measured at 492 nm.

REFERENCES

1. Bøyum,A. (1968). Isolation of mononuclear cells and granulocytes from human blood. Scand.J.Clin.Lab.Invest.21 (Suppl.97),77-89.

2. Nelson,R.D., Quie,P.G. and Simmons,R.L. (1975). Chemotaxis under agarose: A new and simple method for measuring chemotaxis and spontaneous migration of human polymorphonuclear leukocytes and monocytes. J.Immunol. 115,1650-1656.

3. Williams,T.J. and Jose,P.J. (1981) Mediation of increased vascular permeability after complement activation. J.Exp.Med. 153, 136-153.

4. Laemmli.U.K. (1970) Cleavage of structural proteins during assembly of the head of bacteriophage T4. Nature 222, 680.

5. Oi, V.T. and Herzenberg, L.A. (1980) Immunoglobulin-producing hybrid cell lines. In: Mishell, B.B. and Shiigi, S.M. ( Eds.), Selected methods in cellular immunolgy ( W.H. Freeman & Co., San Franciso ) pp. 351-372.

6. Davidson, R.L., O'Malley, K.A. and Wheeler, T.B. (1976) Polyethylene glycol-induced mammalian cell hybridization: Effect of polyethylene glycol molecular weight and concentration. Somat. Cell Genet. 2, 271-280.

7. De Blas, A.L., Ratnaparki, M.V. and Mosiman,J.E.( 1981) Estimation of the number of monoclonal hybridomas in a cell fusion experiment. Effects of post-fusion dilution on hybridoma survival. J. Immunol. Methods 45, 109-115.

## Claims

1. A method of producing monoclonal antibodies against complement factor C5a, which includes the steps of:

a) immune stimulation of spleen cells with C5a;

b) hybridization of the immunized spleen cells and myeloma cells;

c) selection of hybridoma cells for the production of antibodies against C5a; and

d) implantation of the hybridoma cells in the peritoneal cavity of animals of the same species from which the spleen cells and the myeloma cells were isolated, to produce the antibodies.

2. Immunoadsorption material for the binding of complement factor C5a, which comprises a matrix material having bound thereto monoclonal antibodies against C5a.

3. Immunoadsorption material according to claim 2, wherein the matrix material is Sepharose.

4. Immunoadsorption material according to claim 2, wherein the matrix material is in the form of mono-disperse particles.

5. Immunoadsorption material according to claim 4, wherein the particles are polystyrene.

6. A method of removing complement factor C5a from biological fluids, which comprises contacting the biological fluid containing C5a with an immunoadsorption material as claimed in any of claims 2 to 5.

7. A method according to claim 6, wherein the biological fluid is passed through a layer of the said material.

8. A method of detecting and, optionally, quantitatively determining the amount of complement factor C5a in a biological fluid, which comprises the steps of:

a) introducing the biological fluid into a micro-ELISA-well coated with monoclonal antibody against C5a;

b) washing out the cell;

c) introducing into the cell labelled monoclonal antibody against the wall-bound C5a;

d) washing out the cell;

e) detecting, and optionally quantifying, the labelled antibody bound in the cell.

9. A method according to claim 8, wherein the labelled monoclonal antibody is labelled with biotin or an enzyme, and in step (e), the bound labelled antibody is contacted with steptavidin or a substrate for the enzyme label, respectively.

10. Hybridoma cells cloned with genes to indicate the presence of antibodies against C5a.

11. A kit for the detection of C5a in biological fluids, which kit comprises:

a) micro-ELISA-wells coated with monoclonal antibody against C5a;

b) labelled monoclonal antibody (eg. biotin-conjugated or enzyme-conjugated) with specificity for one or more accessible sites on the bound C5a;

c) a substance for reaction with the label, eg. steptavidin if the label is biotin or a substrate if the label is an enzyme.

CHEMOTACTIC INDEX

M NaCl IN THE ELUATE

## Fig. 1

$OD_{492nm}$

□ 5H8B9
▲ 2A5E3
○ 4A3A9
▨ 2A4G7
● 3B5D8
△ 4B1C11

DILUTION

## Fig. 2

Fig. 3

Fig. 4

Fig. 5